## Europäisches Patentamt
## European Patent Office
## Office européen des brevets

(19)

(11) Publication number: **0 059 172**
A1

(12)
# EUROPEAN PATENT APPLICATION

(21) Application number: **82830033.5**

(22) Date of filing: **17.02.82**

(51) Int. Cl.³: **A 61 B 5/04, H 03 F 3/08**

(30) Priority: **19.02.81 IT 1985681**

(43) Date of publication of application: **01.09.82 Bulletin 82/35**

(84) Designated Contracting States: **BE CH DE FR GB LI SE**

(71) Applicant: **Villani, Maria, Via Vallarsa, 24, I-20100 Milano (IT)**

(72) Inventor: **Villani, Maria, Via Vallarsa, 24, I-20100 Milano (IT)**

(74) Representative: **Cicogna, Franco, Ufficio Internazionale Brevetti Dott.Prof. Franco Cicogna Via Visconti di Modrone, 14/A, I-20122 - Milano (IT)**

(54) **Device for detecting bioelectric signals, in particular electrocardiac signals.**

(57) The device comprises transducing electrodes (1,2) for picking up the active currents of the cardiac muscle of a patient, amplifying means (4) for amplifying the picked up signals, a light source (11') effective to provide collimated light beams, a modulating stage (9') cascade coupled to the amplifying means (4) and effective to modulate the light beams from the light source (11') under the control of the amplified electric signals, a fibre optic cable (6) to convey to processing means the modulated light beams, and a d.c current power supply for supplying the amplifying means (4), modulating stage (9') and light source (11').

EP 0 059 172 A1

COMPLETE DOCUMENT

The present invention relates to electrocardiographic apparatus and, more specifically to a device effective to detect the electrocardiographic signals, as picked up by conventional transducer members applied to the patient body, and send said signals, in a noise and spurious signal free form, to a suitable processing apparatus effective to provide a displaying thereof.

As it is well known, the most recent diagnostic and monitoring techniques based on electrocardio-graphic signals provide for the use of a processor or computer means for evaluating the pathologic phenomena, which occur in a long period of time. These systems, more specifically, are designed to detect the cited phenomena and store the related data in suitable memory devices.

Thus a physician is able of examining, at any desired times, the patient conditions determined in a long monitoring period, and recorded in the form of digital data or a diagram.

On the other hand, these known apparatus are affected by several drawbacks, the main thereof is that the processing means, though they are suitably programmed, are hardly able of detecting possible

0059172

pathologic anomalies from the recorded data, since
the latter is affected by disturbances due to electrical
noise, miographic noise or patient movements.

Accordingly the available data is inevitably
altered and are of poor help to the physician

Thus the task of the present invention is
to remarkably improve the reliability of an electro-
cardiographic signal in such a way as to eliminate
therefrom any possible noise, thereby providing such
an apparatus or device effective to send to the signal
processing means truly noise free electric signals
providing the physician with a very accurate information
about the pathologic cardiac phenomena of a patient.

Within the scope of the thereinabove mentioned
task, it is a main object of the present invention
to provide a device, of the mentioned type, which is
structurally very simple, easy to be used and which does
not present any risks for the patient during the period
of use of the device itself.

The aforesaid task and objects, as well as
yet other objects which will become more apparent hereinafter, are achieved by an electrocardiographic signal
detecting device, characterized in that it comprises, in combination,at least a transducer member or electrode
pair,effective to be associated to desired points of
a patient body to transduce the electrocardiographic
signals into electric signals,an amplifying stage
for amplifying the electric signals from said transducer
members,a light source effective to provide collimated
light beams,a modulating stage cascade coupled to
said amplifying stage and effective to modulate said
light source light beams under the control of said
electric signals from said amplifying stage,a fibre
optic cable,including at least a fibre optic,and
coupled to the output of said modulating stage to
convey said modulated light beams or signals to
processing means and a d.c current power supply for
supplying said amplifying stage,modulating stage and
light source.

As a variation the light beam source
can be included in said processing means and the detecting device according to the invention can be supplied
therefrom through a further fibre optic extending from
said processing means.

The present invention will be described thereinafter in a more detailed way,with reference to a preferr- ed embodiment thereof, being illustrated by way of an indicative though not limitative example in the accompanying drawings, where:

fig.1 is a schematic block diagram of the electrocardiographic detecting device or apparatus according to the present invention;

fig.2 is a schematic diagram illustrating a possible embodiment of the amplifying stage associated to the electrocardiographic signal detecting device according to the invention;

fig.3 is a schematic block diagram of the light modulating stage associated to the detecting device according to the invention, provided for one channel operation; and

fig.4 is another schematic block diagram of yet another embodiment of the light modulating stage associated to the detecting device according to the invention, provided for a multiple channel operation.

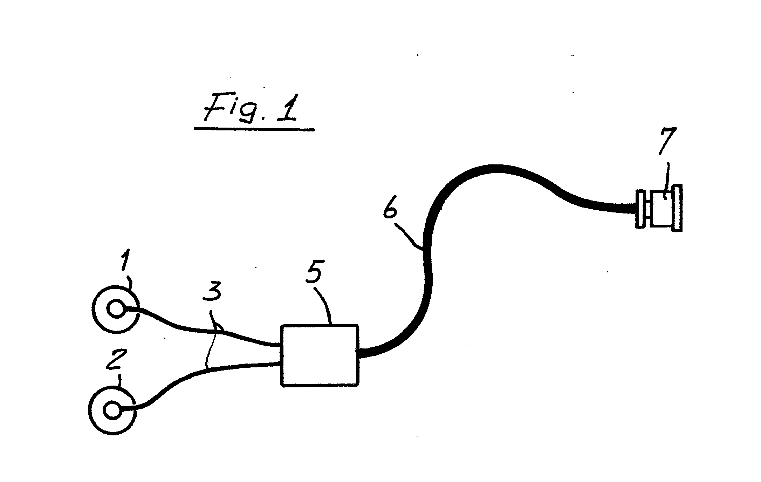

With reference to the figures of the accompanying drawing, the electrocardiographic or electrocardiac signal detecting device according to the present invention

comprises (see fig.1) two transducing members or electrodes 1,2 effective to be associated to the patient body at any desired zone thereof,each said transducing member being electrically coupled,through a wire 3, to a respective amplifying stage 4,which will be described in a more detailed way thereinafter.

Alternatively it is possible to provide a single amplifying stage for all of the transducing members,in which case suitable delay stages will be interposed in order to permit the amplification of the several transducing member signals.

Advantageously,each said amplifying stage,or the single amplifying stage,may consist of a linear integrated circuit amplifier,including,for example, an operational amplifier,operating in the low frequency range,and characterized by a high output current,high open loop gain,very low input current,common mode negative voltage equal to zero,low noise and great insensibility to temperature variations.

The amplifying stages or stage,in particular, as well as the other operating components of the detecting device will be supplied with power from a built-in d.c current source,also included in the main block 5 of the detecting device,such as a conventional small size battery.

According to the present invention, the block 5 also comprises a light source, which latter may consist of a light emitting diode or LED, or a laser diode, also supplied with power by said built-in battery.

The light beams emitted by said light source are modulated by suitable modulating circuitry, which will be described in a more detailed way thereinafter, controlled by the electric signals from said amplifying stage or stages

The modulated light beams at the output of the modulating circuitry or stage are collimated or concentrated at one end of an optic cable 6, including one or more fibre optic, and provided for being coupled to processing means (not shown) through a suitable conventional connector 7.

In said processing means the modulated light beams will be converted again into electric signals, which, upon processing by said processing means, will provide, on suitable display means, information accurately relating to the cardiac activity of the patient.

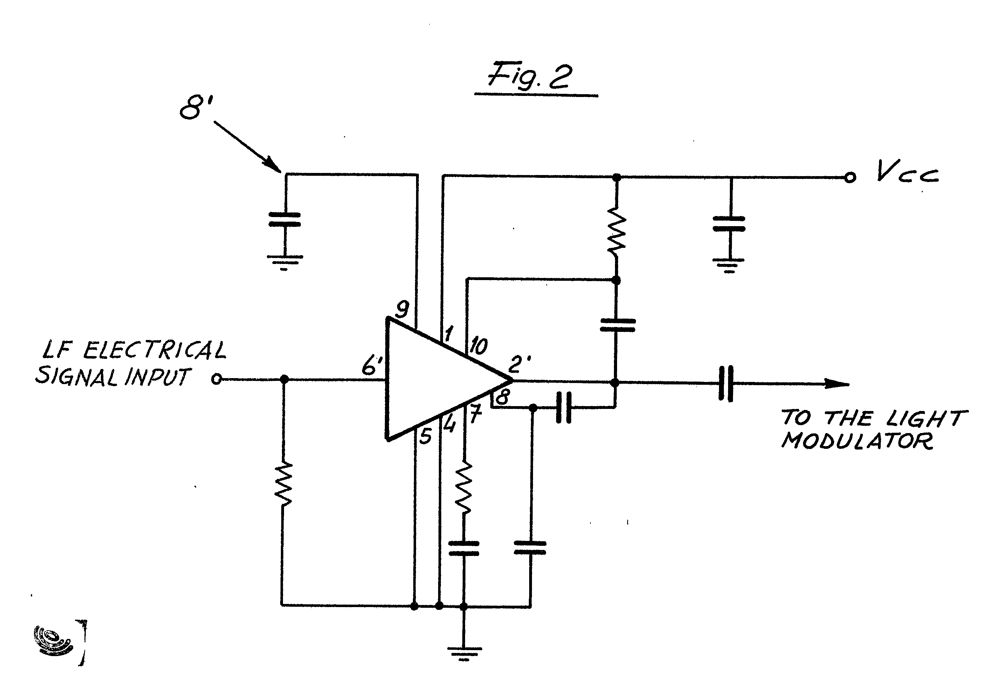

With reference to fig.2, a possible embodiment of an amplifying stage suitable for application in the detecting device according to the invention will be described thereinafter.

As it is shown, said amplifying stage comprises, as the main component thereof an operational amplifier, for example the SF.C 2790 C type, which is commercially available from the firm THOMSON-CSF, Paris, to the pin 6' thereof there is coupled the electric signal from a said transducing member, which variable electric signal is typically a low frequency signal.

The amplified output signal will be taken on the pin 2' of the operational amplifier, indicated overally at 8', and, through suitable impedance matching components, it will drive or control the light modulating stage included in the detecting device according to the invention.

In the case in which a single amlifying stage is used, suitable delay stages will be interposed between the respective transducer members and the pin 6' of said operational amplifier 8'. Alternatively, as thereinabove stated, it is possible to provide a respective amplifyingstage for each transducer.

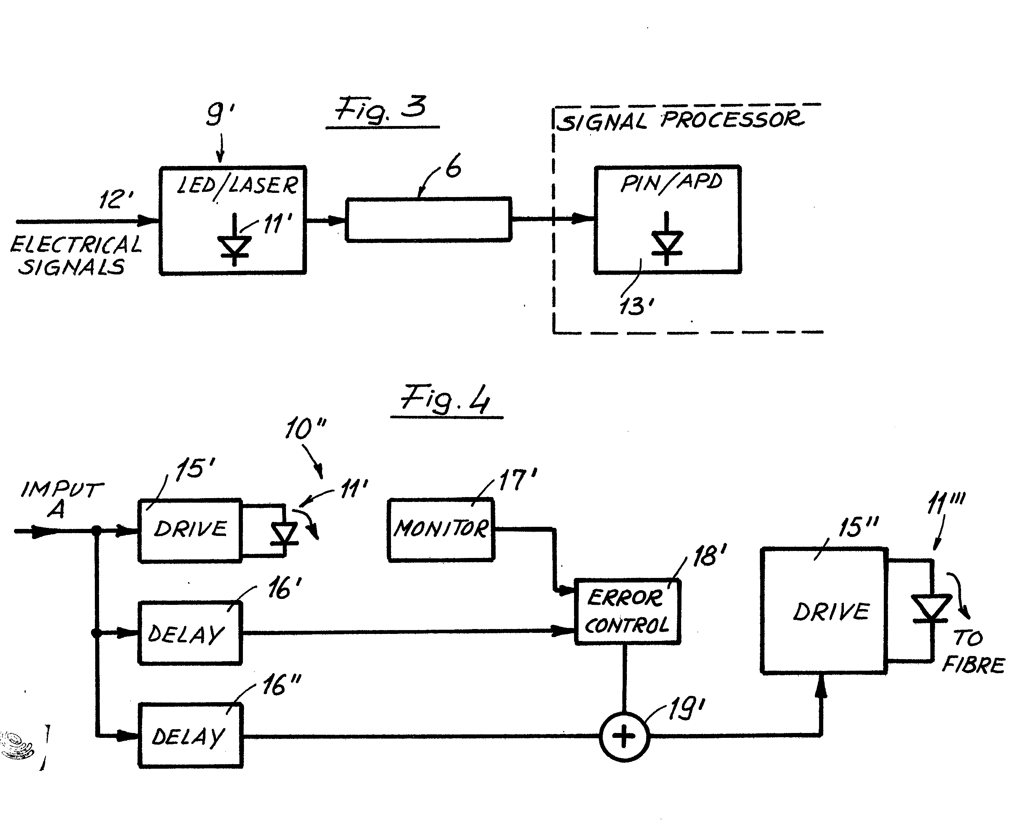

With reference to fig.3 a possible embodiment of the light modulating stage included in the device according to the invention will be briefly described thereinafter.

In fig.3 said modulating stage has been indicated by the reference number 9'.More specifically it comprises,in the block 10' thereof,modulating circuitry,not specifically illustrated since it is well known to those skilled in the art,including as a main component thereof a LED or LASER diode, indicated schematically at 11'.The input of the modulating stage 9' is from a respective transducing member or electrode 1,2 and,in fig.3,it has been indicated at 12' by the arrow.

The output of the modulating stage 9' will be coupled,through said optic cable 6,to the processing means of which only the stage has been illustrated for converting again the light modulated signals on the optic cable 6 into electric signals to be processed for deriving the cardiac activity data.

Also this demodulating stage has been schematically represented by a block 13' and it will comprise,for example,a PIN diode or APD (avalanche photodiode) converter,effective to transform the modulated light beams into related electric signals.

The specific circuitry of this light demodulator has not been shown since it does not form part of the present invention and is ,on the other hand,well known to those skilled in the art.

With reference to fig.4 a possible further embodiment of the light modulating stage included in the detecting device according to the present invention will be described thereinbelow, which embodiment has been provided for a multiple channel operation of the detecting device.

More specifically, in fig.4 the light modulating stage, which has been generally indicated by the reference number 10" comprises two equivalent light emitting diodes or LED's indicated respectively at 11" and 11"'. Each LED comprises its respective driving circuitry, indicated schematically by the blocks 15' and 15". The input of the controlling electric signals from the transducing members is indicated by the arrow A.

In the diagram of fig.4 it should also be noted that delay blocks 16' and 16" has been provided also receiving the input electric signals, as well as a monitoring block 17' and an error control block 18', the output thereof is sent to an error node 19' also receiving the signal from the delay block 16" and controlling the driving block 15" of the second LED 11"'. The operation of this stage will be also obvious to those skilled in the art, thereby it will be not

described therein in further details.On the other hand it should be noted that,with this embodiment of the light modulating device a further compensation of possible non-linerities of the LED's is obtained,thereby the transmission losses are at a minimum which further adds to the accuracy of the detecting device according to the invention.

The operation of the device will be obvious

in the light of the preceding disclosure.

As a variation,instead of the built-in light source,it is possible to locate the latter in the processing means,in which case the beam light will be sent to the modulating stage through a fibre optic included in the optie cable 6.

The invention as disclosed fully achieves the intended objects.In particular it should be pointed out that a detecting device has been provid-

ed which has the following outstanding advantages:

1- a complete elimination of any electric noise from electric apparatus present in the room therein the patient is located,such as an operating theatre,since the wires coupling the sensing electrodes to the device are very short and shielded:in this way there are obtained amplified electric signal accurately indicative of the true cardiac activity of the patient,differently from

the known electrocardiografic apparatus.More specifically in a constructed embodiment of the device the common mode rejection of the overall system (device and coupling cable) was of 160 db.

2- a remarkable reduction of the myographic noise,as only two electrodes are used,due to a very high common mode impedance level,mainly with respect to the capacitive component

3- a remarkable reduction of the noise due to possible movements of the patient,because of those same reasons thereinabove cited.

4- an absolute safety against risks of microshocks and macroshocks,which are very frequent as other apparatus are operating on the same patient.

5- very low transmission losses,due to the use of a fibre optic cable.

Though the detecting device according to the invention has been thereinabove illustrated with reference to a preferred embodiment thereof,it should be noted that it is susceptible to several modifications and variations all falling within the scope of the invention.

Thus,for example,it may include more than two sensing electrodes and be used in applications different from an electrocardiographic application.Moreover it may be associated to any suitable recording output device.

C L A I M S

1- An electrocardiographic or electrocardiac signal detecting device, characterized in that it comprises, in combination, at least a transducer member(1,2) or electrode pair, effective to be associated to desired points of a patient body, an amplifying stage (4) for amplifying the electric signals from said transducing members (1,2), a light source (11') effective to provide collimated light beams, a modulating stage (9') cascade coupled to said amplifying stage (4) and effective to modulate said light source (11') light beams under the control of said electric signals from said amplifying stage, a fibre optic cable (6), including at least a fibre optic, and coupled to the output of said modulating stage (9') to convey said modulated light beams or signals to processing means and a d.c current power supply for supplying said amplifying stage (4), modulating stage (9') and light source (11').

2- A device according to claim 1, characterized in that it comprises a respective amplifying stage (4) and a respective modulating stage (9') for each said transducing member (1,2).

BAD ORIGINAL

3- A device according to claim 1, characterized in that it comprises a single amplifying stage (4) and a single modulating stage (9' or 10") for all or said transducing members (1,2).

4- A device according to claim 1, characterized in that said light source includes at least a LED or LASER diode (11')

5- A device according to claim 1, characterized in that said amplifying stage (4) includes at least an operational amplifier.

6- A device according to claim 1, characterized in that it is provided for cooperating with a light demodulating device including a PIN or avalanche diode.

7- A device according to claim 1, characterized in that said light source is included in said processing means.

8- A device according to claim 1, characterized in that said amplifying stage, light source and modulating stage (9') are all supplied by said d.c current power supply consisting of a conventional battery and are all contained in the same housing.

9- An electrocardiographic or the like apparatus including a device according to any preceding claims.

Fig. 1

*Fig. 2*

LF ELECTRICAL
SIGNAL INPUT

Vcc

TO THE LIGHT
MODULATOR

8'

9  1  10  2'  8  6'  5  4  7

0059172

Fig. 3

Fig. 4

0059172

Application number

EP 82 83 0033

# European Patent Office

## EUROPEAN SEARCH REPORT

## DOCUMENTS CONSIDERED TO BE RELEVANT

| Category | Citation of document with indication, where appropriate, of relevant passages | Relevant to claim |
|---|---|---|
| X | THE ELECTRONIC ENGINEER (US), vol. 29, no. 12, December 1970; RADNOR (US) "Biomedical Testing", page 53 <br><br> * Page 53 * | 1,4 |
| Y | MEDICAL & BIOLOGICAL ENGINEERING, vol. 6. no. 4, August 1968; STEVENAGE/HERTS (GB) H. VAN DER WEIDE et al: "A photon-coupled Amplifier for the trans-mission of physiological signals", pages 447 and 448 and 448A <br><br> * Pages 447-448A * | 1,6,8 |
| Y | DE - A - 2 328 834 (MARQUETTE ELECTRONICS, INC.) <br><br> * Page 6, line 18 - page 7, line 10; page 8, line 4 - page 9, line 10; page 10, line 9 - page 11, line 24; figure 1 * | 1,2,4 |
| Y | IEEE TRANSACTIONS ON BIO-MEDICAL ENGINEERING, vol. BME-18, no. 6, November 1971; NEW YORK (US) W.P. HOLSINGER et al: "Patient Electrode Isolation Adapter", page 428-430 <br><br> * Page 428-430 * | 1,4,5, 8 |

### CLASSIFICATION OF THE APPLICATION (Int. Cl. 3)

A 61 B 5/04
H 03 F 3/08

### TECHNICAL FIELDS SEARCHED (Int.Cl. 3)

A 61 B 5/04
H 03 F 3/08

### CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

X | The present search report has been drawn up for all claims

| Place of search | Date of completion of the search | Examiner |
|---|---|---|
| The Hague | 27-04-1982 | RIEB |

EPO Form 1503.1 06.78

European Patent Office

**EUROPEAN SEARCH REPORT**

| | **DOCUMENTS CONSIDERED TO BE RELEVANT** | | CLASSIFICATION OF THE APPLICATION (Int. Cl.³) |
|---|---|---|---|
| Category | Citation of document with indication, where appropriate, of relevant passages | Relevant to claim | |
| Y | US - A - 3 808 502 (A.J. BABILIUS/ THE BIRTCHER CORP.)<br><br>* Abstract; column 4, line 41 - column 5, line 32; column 8, lines 1-18; figures 2,3,5 *<br><br>---- | 1,3-5 | |
| | | | **TECHNICAL FIELDS SEARCHED (Int. Cl.³)** |

EPO Form 1503.2  06.78